# EUROPEAN PATENT SPECIFICATION

(11) **EP 1 438 410 B1**
(45) Date of publication and mention of the grant of the patent: **14.12.2005**
(21) Application number: 02777445.4
(22) Date of filing: 25.10.2002
(51) Int. Cl.: C12N 15/63, C12N 15/85, C12N 15/12, C12N 15/54, C12N 5/10, A61K 35/50, C07K 14/82, C12N 9/12

(54) **PROMOTERS TO CONTROL CELL DIFFERENTIATION**
PROMOTOREN ZUR KONTROLLE DER ZELLDIFFERENZIERUNG
PROMOTEURS DESTINES A REGULER LA DIFFERENCIATION CELLULAIRE

(30) Priority: 26.10.2001 GB 0125773
(43) Date of publication of application: 21.07.2004
(73) Proprietor: Reneuron Limited, Guildford, Surrey GU2 7AF (GB)
(72) Inventor: SINDEN, John, ReNeuron Limited, Guildford,Surrey GU2 7AF (GB); DONG, Ziping, ReNeuron Limited, Guildford, Surrey GU2 7AF (GB)
(74) Representative: Jappy, John William Graham
(86) International application number: PCT/GB2002/004828
(87) International publication number: WO 2003/035879

(56) References cited:
- WO-A-01/21790
- WO-A-01/36482
- WO-A-01/66781
- WO-A-96/24669
- WO-A-97/10329
- WO-A-98/37181
- US-B1- 6 303 370
- LOTHIAN C ET AL: "IDENTIFICATION OF BOTH GENERAL AND REGION-SPECIFIC EMBRYONIC CNS ENHANCER ELEMENTS IN THE NESTIN PROMOTER" EXPERIMENTAL CELL RESEARCH, SAN DIEGO, CA, US, vol. 248, no. 2, 1 May 1999 (1999-05-01), pages 509-519, XP000998216 ISSN: 0014-4827 cited in the application
- LOTHIAN C ET AL: "AN EVOLUTIONARILY CONSERVED REGION IN THE SECOND INTRON OF THE HUMAN NESTIN GENE DIRECTS GENE EXPRESSION TO CNS PROGENITOR CELLS AND TO EARLY NEURAL CREST CELLS" EUROPEAN JOURNAL OF NEUROSCIENCE, OXFORD UNIVERSITY PRESS, GB, vol. 9, no. 3, March 1997 (1997-03), pages 452-462, XP000998218 ISSN: 0953-816X cited in the application
- ZIMMERMAN L ET AL: "INDEPENDENT REGULATORY ELEMENTS IN THE NESTIN GENE DIRECT TRANSGENEEXPRESSION TO NEURAL STEM CELLS OR MUSCLE PRECURSORS" NEURON, CAMBRIDGE, MA, US, vol. 12, no. 1, January 1994 (1994-01), pages 11-24, XP000998222
- ANDRESSEN CHRISTIAN ET AL: "Nestin-specific green fluorescent protein expression in embryonic stem cell-derived neural precursor cells used for transplantation" STEM CELLS, ALPHAMED PRESS, DAYTON, OH, US, vol. 19, no. 5, 2001, pages 419-424, XP002197255 ISSN: 1066-5099

## Description

### Field of the Invention

The present invention relates to the use of specific regulatory promoters to control differentiation in mammalian cells.

### Background of the Invention

There is a growing awareness and understanding of the importance of transplantation therapy to treat damage to tissues and organs. While organ transplantation is widely practiced, therapies based on the transplantation of individual cells are still in a relatively early phase of clinical development.

For example, there is growing recognition that the transplantation of suitable cells into a damaged brain may improve or correct any sensory, motor, behavioral or psychological deficits caused by the damage.

For cell-based therapies to be useful, it must be possible to obtain sufficient cells for transplantation. One means for ensuring this is to culture undifferentiated cells under conditions which allow repeated cell division and growth. One difficulty with using undifferentiated cells is that unregulated cell division must be switched off either prior to or on transplantation into the patient, to prevent uncontrolled growth at the site of transplantation.

Many different techniques have been developed to provide suitable cells for transplantation. With regard to neural transplantation, one approach has been to maintain undifferentiated foetal cells under culture conditions that permit cell division to occur, and to subsequently induce differentiation *in vitro,* prior to transplantation.

Reynolds and Weiss, Science, 1992;255:1707, disclose the use of epidermal growth factor (EGF) to induce the in vitro proliferation of adult mouse brain cells. Under suitable conditions it was thought that the cells could be induced to differentiate into astrocytes and neurons.

International Patent Application No. WO-A-94/16059 discloses a technique for maintaining a primary neuronal /cell culture *in vitro* by culturing the cells in a serum-free media supplemented with at lease one trophic factor.

International Patent Application No. WO-A-97/10329 discloses an alternative technique, using a conditionally-immortalized cell line. This cell line comprises an immortalizing temperature-sensitive oncogene which, under permissive conditions, maintains neuroepithelial stem cells in the undifferentiated state. Upon transplantation the oncogene is switched off due to the higher temperature of the human body (37°C) and the cells differentiate into the cell types required to repair damage. The advantage of using the oncogene is that the cells are maintained in the undifferentiated state until transplantation, at which point the cells differentiate, in response to the specific damage, into the phenotype of the damaged or lost cells. US 5688692 also discloses cells expressing a non-DNA-binding, temperature-sensitive T antigen.

WO-A-01/21790 discloses that cells transduced with a conditionally-inducible oncogene and at least the catalytic subunit of the telomerase complex are immortal under permissive conditions but lose immortality under non-permissive conditions.

Nestin is an intermediate filament protein. Nestin expression has been used extensively as a marker for stem cells and progenitor cells in the central nervous system. The down-regulation of nesting *in vivo* correlates with the differentiation of neural stem cells.

US6,303,370 discloses nucleic acid constructs comprising regulatory elements from the second intron of the rat nestin gene in combination with selected nucleic acid sequences, that are useful for tissue-specific or temporal expression of the selected nucleic acid sequence.

Andressen et al, Stem Cells, 2001; 19(5) :419-24 discloses that expression of enhanced green fluorescent protein under control of a thymidine kinase promoter/nestin second intron was detected in nestin immunoreactive neural precursor cells after selection of murine embryonic stem cells in chemically defined medium.

### Summary of the Invention

The present invention is based on the understanding that the regulatory elements that control nestin expression can be utilised to regulate expression in cells transected with oncogenes.

According to a first aspect of the invention, a recombinant construct comprises a polynucleotides that encodes a conditionally-inducible oncogene operably linked to the enhancer element of the second intron of the nestin gene, or a functional fragment thereof.

According to a second aspect, a cell comprises a construct as defined above.

According to a third aspect, the constructs of the invention are used to conditionally immortalise a cell *in vitro.*

According to a fourth aspect, the cells of the invention are used in the manufacture of a medicament for transplantation to treat a disease caused by cell loss or damage.

The enhancer is functional when the host cell is in the undifferentiated state, and so cell proliferation proceeds by the action of the oncogene. When the cell environment is switched to non-permissive conditions, i.e. elevated temperature, the oncogene is not, or weakly, expressed and the cell differentiates. The enhancer is also non-functional on differentiation, and this provides a further mechanism preventing unregulated expression of the oncogene.

### Brief Description of the Drawings

The invention is described with reference to the accompanying drawings, wherein:

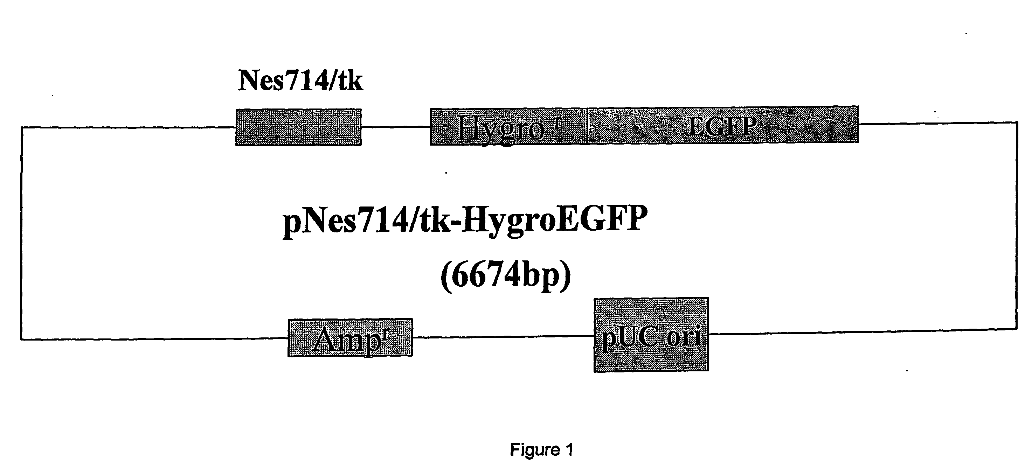

Figure 1 is the pNes714/tk-HygroEGFP construct where a human nestin promoter containing the 714bp fragment of the nestin secondary intron is followed by the 160bp of the basic HSV tk promoter with a HygroEGFP gene acting as a reporter gene, and a selection marker;

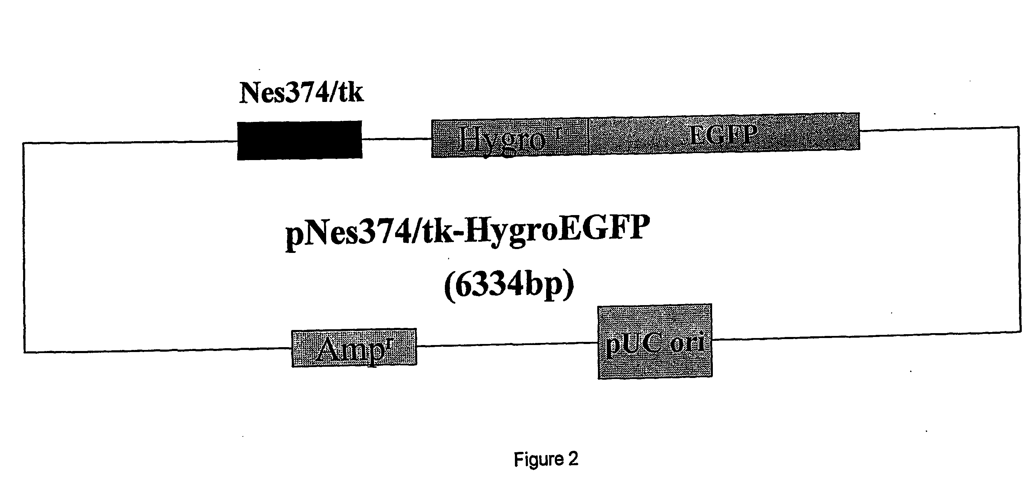

Figure 2 is the pNes374/tk-HygroEGFP construct where a human nestin promoter containing the 374bp fragment of the nestin secondary intron is followed by the 160bp of the basic HSV tk promoter, a HygroEGFP gene acts as a report gene, and a selection marker;

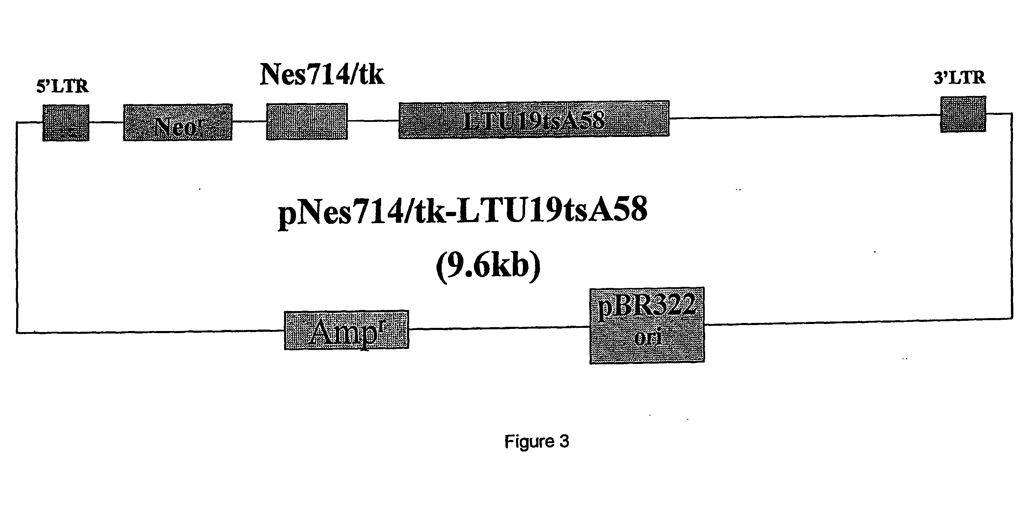

Figure 3 is the viral construct of pNes714/tk-LTU19tsA58 where the U19tsA58 mutation of Large T gene is driven by the nestin promoter Nes714/tk, the neomycin resistance gene is driven by a Long Terminal Repeat LTR, the expression of the large T protein is controlled by a regulatory promoter of Nes714/tk and a temperature-sensitive gene of the U19tsA58;

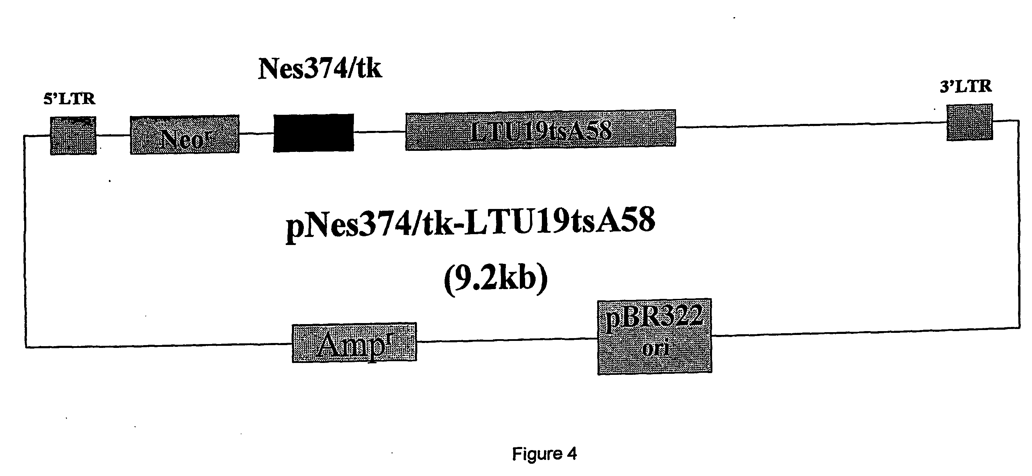

Figure 4 is the viral construct of pNes374/tk-LTU19tsA58 where the U19tsA58 mutation of the Large T gene is driven by the nestin promoter Nes374/tk, the neomycin resistance gene is driven by the Long Terminal Repeat LTR, the expression of the large T protein is controlled by a regulatory promoter of Nes374/tk and a temperature-sensitive gene of the U19tsA58;

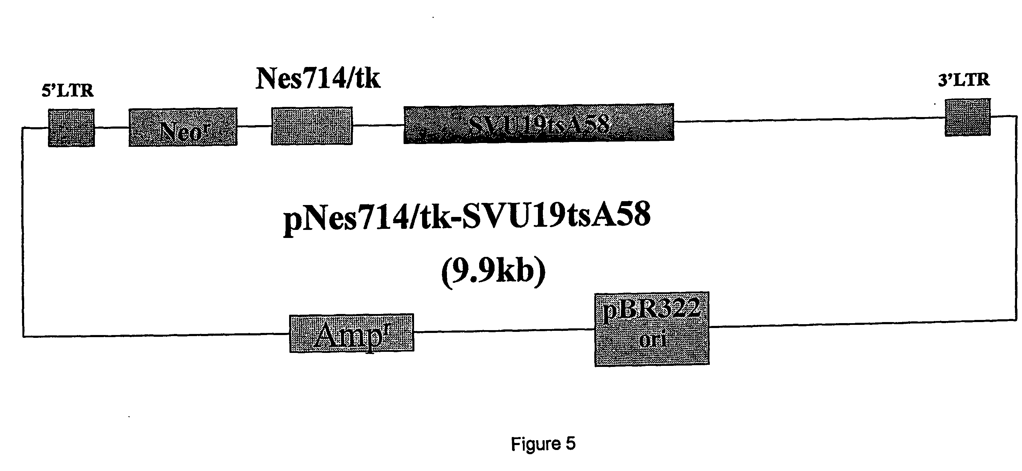

Figure 5 is the viral construct of pNes714/tk-SVU19tsA58 where the U19tsA58 mutation of the SV40gene is driven by the nestin promoter Nes714/tk, the neomycin-resistance gene is driven by the Long Terminal Repeat LTR, the expression of the SV40 gene is controlled by a regulatory promoter of Nes714/tk, and a temperature-sensitive gene of the U19tsA58 controls the large T protein expression;

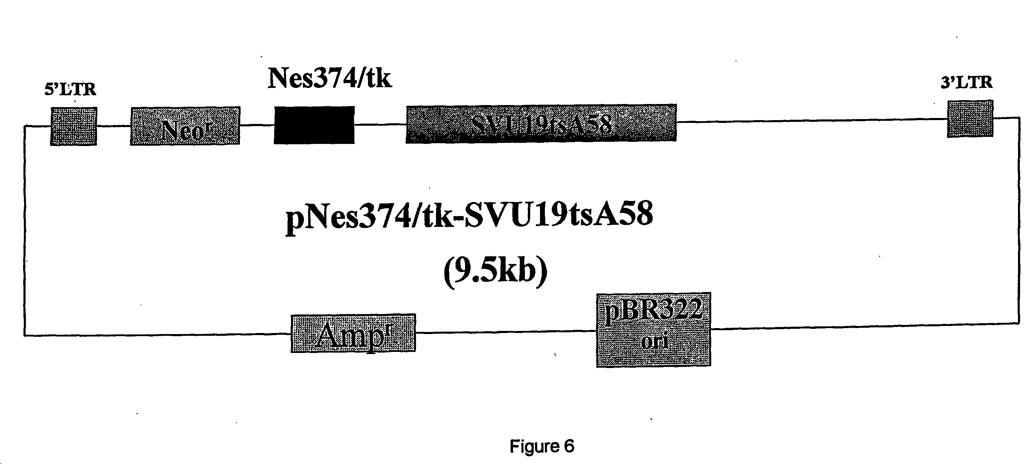

Figure 6 is the viral construct of pNes374/tk-SVU19tsA58 where the U19tsA58 mutation of the SV40 gene is driven by the nestin promoter Nes374/tk, the neomycin-resistance gene is driven by the Long Terminal Repeat LTR, the expression of the SV40 gene is controlled by a regulatory promoter of Nes374/tk, and a temperature-sensitive gene of the U19tsA58 controls the large T protein expression;

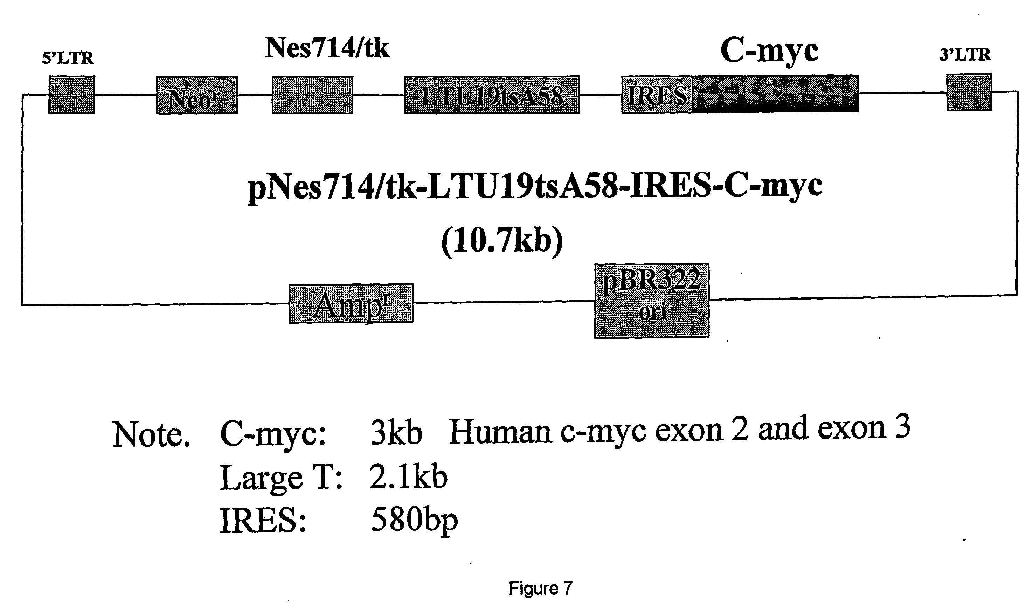

Figure 7 is the viral construct of pNes714/tk-LTU19tsA58-IRES-C-myc where the nestin promoter Nes714/tk drives both a mutated large T gene LTU19tsA58 and a C-myc gene, an internal ribosome entry site (IRES) is placed between the two genes and the neomycin-resistance gene is driven by a Long Terminal Repeat LTR;

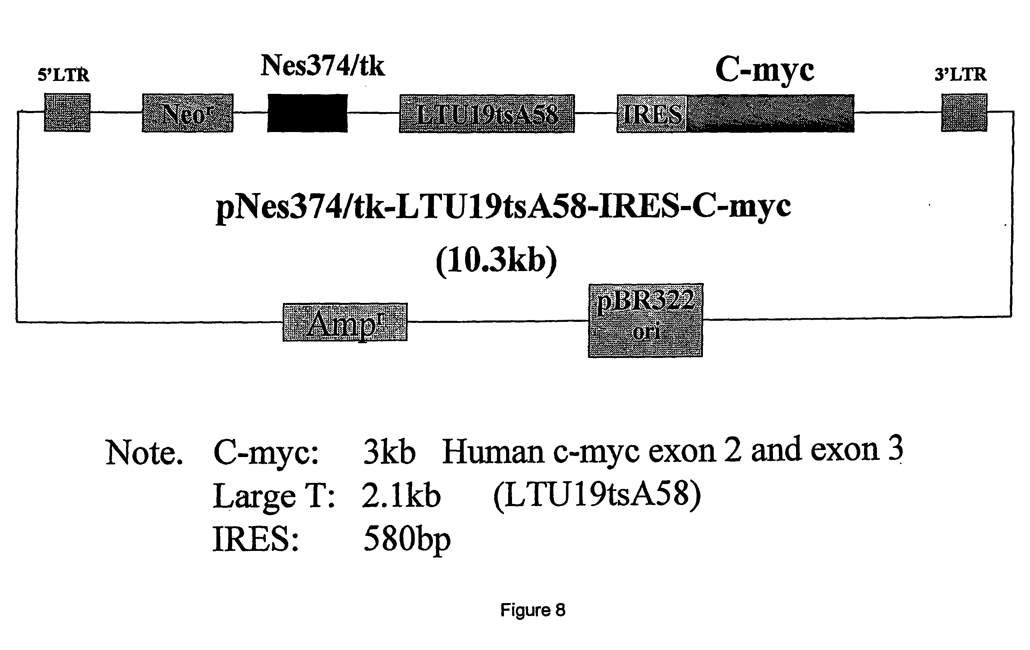

Figure 8 is the viral construct of pNes374/tk-LTU19tsA58-IRES-C-myc where the nestin promoter Nes374/tk drives both a mutated large T gene LTU19tsA58 and a C-myc gene, an internal ribosome entry site (IRES) is placed between the two genes and the neomycin-resistance gene is driven by a Long Terminal Repeat LTR;

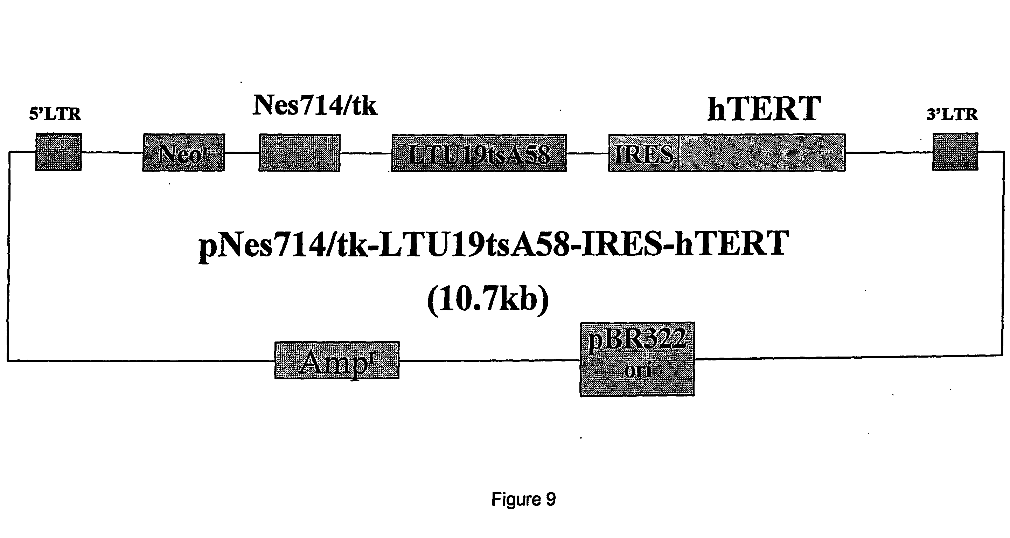

Figure 9 is the viral construct of pNes714/tk-LTU19tsA58-IRES-hTERT where the nestin promoter Nes714/tk drives both a mutated large T gene LTU19tsA58 and an hTERT gene, an internal ribosome entry site (IRES) is placed between the two genes and the neomycin-resistance gene is driven by a Long Terminal Repeat LTR;

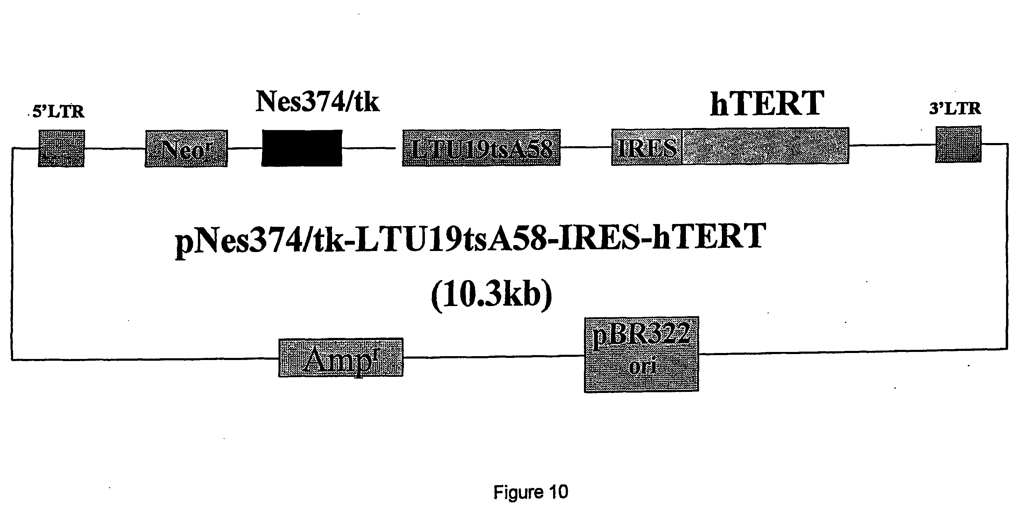

Figure 10 is the viral construct of pNes374/tk-LTU19tsA58-IRES-hTERT where the nestin promoter Nes374/tk drives both a mutated large T gene LTU19tsA58 and an hTERT gene, an internal ribosome entry site (IRES) is placed between two genes, and the neomycin-resistance gene is driven by a Long Terminal Repeat LTR;

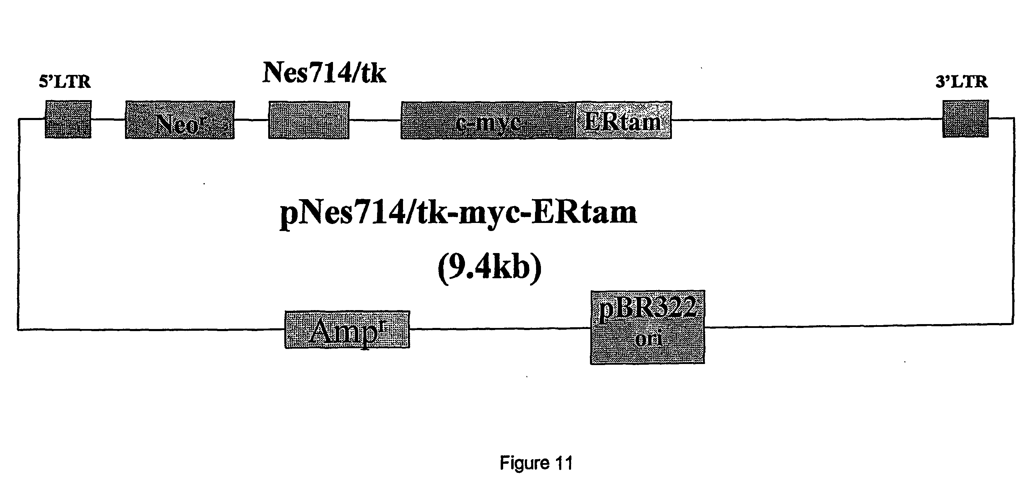

Figure 11 illustrates the construct pNes714/tk-myc-ERtam, where the nestin promoter Nes 714/tk is used to drive expression of c-myc and an estrogen receptor gene ERtam; and

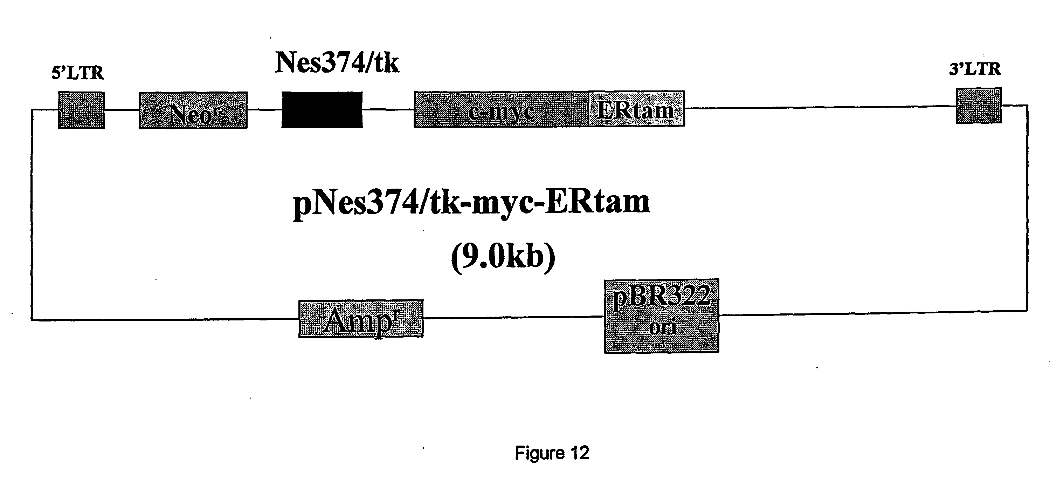

Figure 12 illustrates the construct pNes374/tk-myc-ERtam, where the nestin promoter Nes374/tk is used to drive expression of c-myc and an estrogen receptor gene ERtam.

### Description of the Invention

The present invention discloses methods for preparing cells which are suitable for transplantation therapy and which are immortal up to the time of transplantation.

In general, the preparation of the cells, including the preparation of genetic constructs to be included in the cells, is based on conventional techniques known to those skilled in the art. Suitable methods are also disclosed in Sambrook *et al,* Molecular Cloning, A Laboratory Manual (1989), and Ausubel *et al,* Current Protocols in Molecular Biology (1995), John Wiley & Sons Inc.

The cells according to the invention require a conditionally-inducible oncogene to be present. The term "conditionally-inducible" is used herein to refer to oncogenes, the expression of which can be regulated under certain conditions. The oncogene will undergo expression when so-called permissive conditions are applied. For example, some oncogenes are temperature-sensitive and are only expressed when the temperature of their environment is below a certain value. The oncogenes are therefore not unregulated, and can be switched on and off, depending on the environmental conditions. In one embodiment of the invention, the oncogene that is used is a non-DNA-binding, temperature-sensitive, mutant of the SV-40 large T-antigen gene (U19tsA58) . Suitable alternatives are also known and include the oncogene of the polyoma T-antigen.

Other methods for preparing "conditionally-inducible" oncogenes are well-known in the art and include, for example, the fusion of the c-myc oncogene with various forms of the estrogen receptor (ER) gene. In one example (Littlewood *et al.* Nucleic Acids Res. , 1995; 23 (10) : 1686-90), the human c-myc gene is fused with the 4-hydroxytamoxifen-responsive mutant murine estrogen receptor gene (myc-ERtam) . The fusion protein is only activated when 4-hydroxytamoxifen is present.

The oncogene is comprised on a recombinant DNA or retroviral vector or construct used to transduce/infect the cells. The terms "vector" and "construct" are used interchangeably herein. The vectors or constructs of the invention further comprise the enhancer element of the second intron of the nestin gene. The enhanc er and oncogene are operably linked so that the enhancer provides control over the expression of the oncogene. The enhancer is therefore preferably located upstream of the oncogene sequence.

The enhancer is preferably that corresponding to the human enhancer. In a further preferred embodiment the enhancer is either the 714bp or 374bp enhancer element of the 3' portion of the second intron of the human nestin gene. These are identified in Lothian *et al,* Exp.. Cell Res., 1999; 248: 509-519. Suitable enhancers are also disclosed in Yasorsky *et al,* Developmental Biology, 1999; 205: 309-321 and Muller *et al,* Development, 1998; 125 (16) : 3087-3100.

Functional fragments of the enhancer element are also within the scope of the invention. The term "functional fragment" means that a portion of the enhancer is present, which can still exert a regulatory effect. The use of the term "fragment" includes substitutions or additions to the sequence of the enhancer. For example, single base substitutions may be made without altering the ability of the enhancer to exert a regulatory effect. Suitable fragments will have the ability to hybridise to the complement of the enhancer sequence under stringent hybridisation condition and also exert a regulatory effect.

The "stringent hybridisation condition" are overnight incubation at 42°C in a solution comprising: 50% formamide, 5 x SSC (150 mM NaCl, 15 mM trisodium citrate), 50 mM sodium phosphate (pH 7.6), 5 x Denhardt's solution, 10% dextran sulphate, and 20 *µ*g/ml denatured sheared salmon sperm DNA, followed by washing the filters in 0.1 x SSC at about 65°C.

The cells may also use a regulatable system at the protein level. The Myc-ERtam construct is the human c-Myc protein fused with 4-hydroxytamoxifen responsive mutant murine estrogen receptor. The fusion protein is only activated when 4-hydroxytamoxifen is presence. Additional regulatory element Nes714/tk or Nes374/tk will be located upstream of the myc-ERtam gene. The dual control of gene expression and protein regulation enhances the tight control of immortalisation of the cells.

The cells may also comprise an exogenous polynucleotide that encodes at least the catalytic sub-unit of the telomerase complex. The term "exogenous" is used herein in its normal context to refer to the polynucleotide introduced into the cell, and distinguish from naturally-occurring endogenous polynucleotides. The catalytic sub-unit of the telomerase complex is an enzyme that acts like a reverse transcriptase, and is known in the art. The human sub-unit is disclosed in GB-A-2317891. Additional regulatory elements may also be present. For example, additional promoters may be located on the construct. A promoter may be present to regulate expression of the telomerase gene, or may be present as part of the construct to aid expression of the oncogene.

Regulation of expression may be carried out by methods known to the skilled person. For example, regulation may be effected using the long terminal repeat (LTR) promoter. Alternative promoters will be apparent to the skilled person. For example, regulation may be effected using the cytomegalovirus (CMV) promoter. The CMV promoter is a very strong promoter, and may be preferred when the cells are neural cells, e.g. neuroepithelial stem cells.

Methods for introducing suitable constructs into cells, are known to the skilled person.

Any mammalian cell may be used in the present invention. For example, the cell may be an endothelial cell, and may be used for the revascularisation of the leg, heart and other organs. Preferably, the cell is a human somatic cell, e.g. human epithelial stem cell, which is capable of differentiation into a specific cell type. A particularly preferred cell is an undifferentiated (or precursor) human neuroepithelial pluripotent cell which may be used in neural transplantation to repair cell loss or damage and correct behavioral or psychological deficits. Alternatively, the cell may be a differentiated cell, e.g. the β cells of Islets of Langerhans. Additional cells may include, but are not limited to, those obtainable from the endocrine glands, retinal cells, pancreatic cells, cochlear cells, liver cells, osteoblast and osteoclasts, myoblasts and keratinocytes.

Preferably, the constructs are incorporated into the cell during the early culture phase, usually within the first 10 cell divisions.

The transduced or infected cells may be cultured under conditions known to those skilled in the art. It is preferable that the cells are cultured under non-stressed conditions. A skilled person will appreciate the conditions suitable for each particular cell type, based on conventional culture techniques.

The recombinant cells of the invention may have use in therapy. Methods for the preparation of formulations for delivery to a patient will be apparent to the skilled person. Suitable excipients, diluents etc, will again be apparent based on current practice in preparing cell-based therapies. The amount of cells required for delivery will vary depending on the form of treatment, the severity of the disease/damage, and the need for applying multiple doses over a treatment period. However, the skilled person can readily determine the appropriate treatment based on existing cell transplantation therapies.

The following Example further illustrates the invention.

### Example

In this Example, the regulation of Nes714/tk and Nes374/tk promoters in neural precursor cells is provided.

Nes714/tk and Nes374/tk promoters were constructed with green fluorescent protein (GFP) acting as a reporter gene, and hygromycin-resistance gene (Hygro^{r}) functions as a selection marker (see Figure 1 and Figure 2). Nes714/tk and Nes374/tk fragments were isolated from pNes714/tk-LacZ and pNes374/tk-LacZ constructs (Lothian *et al.*, Eur. J. Neurosci, 1997; 9: 452-462 and Lothian, et al., 1999, supra) by Not I and Hind III restriction digestion. The pHygEGFP construct (Clontech) was digested with Bg1 II and Nhe I to remove the CMV promoter. All three fragments, Nes714/tk, Nes374/tk and pHygEGFP were treated with T4 DNA polymerase to generate blunt-ends. pNes714/tk-HygroGFP and pNes374/tk-HygroEGFP constructs were generated by ligating Nes714/tk and Nes374/tk fragments into the blunt-end (Bg1 II and Nhe I sites) of pHygEGFP.

Constructs of the pNes714/tk-HygroGFP and pNes374/tk-HygroGFP were transfected into conditionally immortalized human neural precursor cell lines generated as described in WO-A-97/10329, grown at the permissive temperature, 33°C. Transfected cells were subsequently selected under hygromycin to generate human neural precursor cell lines contianing pNes714/tk-HygroGFP and pNes374/tk-HygroGFP constructs. Pure GFP positive cells were cultured for 3-7 days at 37°C (with or without 10% serum) for a differentiation assay and at 33°C (control). At 37°C, the human neural precursor cell lines start to differentiate and down-regulation of GFP in these cells was observed, indicating that the pNes714/tk and pNes374/tk promoters are regulated during the transition of human neural precursor cells into the differentiated state. The Nes714/tk and Nes374/tk promoters therefore serve as inducible promoters that drive gene expression exclusively in neural precursor cells, but not in differentiated cells.

A recent study has indicated that no expression in CNS *in vivo* was seen in the Nes714tk/lacZ mice, however, expression of the reporter gene was detected when CNS stem cells were cultured *ex vivo* (J. Neurosci Res, 2002; 15: 784-794). It is possible to expect further down-regulation of GFP when nes714 tk-HygroGFP is used *in vivo.* The difference between in vitro and *in vivo* regulation of Nes714tk may give further advantage for the therapeutic application of the described Nestin enhancer regulation system.

In another aspect of the invention, the Nes714/tk and Nes374/tk promoters were used to construct various viral constructs such as pNes714/tk-LTU19tasA58, pNes374/tk-LTU19tsA58, pNes714/tk-SVU19tsA58, pNes374/tk-SVU19tsA58, pNes714/tk-LTU19tsA58-IRES-C-myc, pNes374/tk-LTU19tsA58-IRES-C-myc, pNes714/tk-LTU19tsA58-IRES-hTERT and pNes374/tk-LTU19tsA58-IRES-hTERT, pNes714/tk-myc-ERtam and pNes374/tk-myc-ERtam (Figures 3 to 12). The genes, LTU19tsA58, SVU19tsA58, C-myc, myc-ERtam and hTERT used in these constructs are used as the immortalising genes. LTU19tsA58 refers to the temperature-sensitive Large T gene. SVU19tsA58 refers to a temperature-sensitive SV40 gene. hTERT refers to the catalytic subunit of the human telomerase reverse transcriptase. Myc-ERtam refers to c-Myc fused with 4-hydroxytamoxifen-responsive mutant murine estrogen receptor domain.

The constructs provide dual control of gene expression via both a regulatory promoter and a temperature-sensitive mutation of the gene or a pharmaceutically inducible gene. In some constructs, two genes, e.g. LTU19tsA58 and C-myc or LTU19tsA58 and hTERT, were used. In this case, LTU19tsA58 acts as an immortalising gene while C-myc and hTERT function as genes which stabilize the chromosome.

## Claims

1. A construct comprising a polynucleotide that encodes a conditionally-inducible oncogene operably linked to the enhancer element of the second intron of the nestin gene, or a functional fragment thereof.

2. A construct according to claim 1, wherein the element is either the 714bp or 374bp element of the 3' portion of the second intron of the human nestin gene.

3. A construct according to claim 1, wherein the oncogene is a c-myc or the temperature-sensitive mutant of the SV40 large T-antigen.

4. A construct according to any preceding claim, further comprising a polynucleotide that acts as a promoter of the oncogene.

5. A construct according to claim 4, wherein the promoter is the HSV tk promoter.

6. A construct according to any preceding claim, further comprising a polynucleotide that encodes the catalytic sub-unit of the telomerase complex.

7. A construct according to any of claims 1 to 5, further comprising a C-myc oncogene.

8. An isolated cell comprising a construct according to any preceding claim.

9. An isolated cell according to claim 8, which is a mammalian undifferentiated pluripotent cell.

10. A cell according to claim 8 or claim 9, which is an undifferentiated, pluripotent human neuroepithelial cell.

11. A cell according to any of claims 8 to 10, for therapeutic use.

12. Use of a construct according to any of claims 1 to 7, to conditionally immortalise a cell in vitro.

13. Use according to claim 12, wherein the cell is as defined in claim 9 or claim 10.

14. Use of a cell according to any of claims 8 to 10, in the manufacture of a medicament for transplantation to treat a disease caused by cell loss or damage.

## Patentansprüche

1. Konstrukt, umfassend ein Polynukleotid, das für ein bedingt induzierbares Onkogen oder ein funktionelles Fragment davon kodiert, welches in funktionsfähiger Weise mit dem Enhancer-Element des zweiten Introns des Nestin-Gens verknüpft ist.

2. Konstrukt nach Anspruch 1, wobei das Element entweder das 714-Bp- oder das 374-Bp-Element des 3'-Teils des zweiten Introns des humanen Nestin-Gens ist.

3. Konstrukt nach Anspruch 1, wobei das Onkogen ein c-myc oder eine temperaturempfindliche Mutante des SV-40-T-Angtigens ist.

4. Konstrukt nach einem der vorhergehenden Ansprüche, das weiter ein Polynukleotid umfasst, das als Promotor des Onkogens wirkt.

5. Konstrukt nach Anspruch 4, wobei der Promotor der HSV-tk-Promotor ist.

6. Konstrukt nach einem der vorhergehenden Ansprüche, das weiter ein Polynukleotid umfasst, das für die katalytische Untereinheit des Telomerase-Komplexes kodiert.

7. Konstrukt nach einem der Ansprüche 1 bis 5, das weiter ein c-myc-Onkogen umfasst.

8. Isolierte Zelle, umfassend eine Konstrukt nach einem der vorhergehenden Ansprüche.

9. Isolierte Zelle nach Anspruch 8, die eine undifferenzierte, pluripotente Säugerzelle ist.

10. Zelle nach Anspruch 8 oder Anspruch 9, die eine undifferenzierte, pluripotente humane neuroepitheliale Zelle ist.

11. Zelle nach einem der Ansprüche 8 bis 10 zur therapeutischen Verwendung.

12. Verwendung eines Konstrukts nach einem der Ansprüche 1 bis 7 zur bedingten Immortalisierung einer Zelle in vitro.

13. Verwendung nach Anspruch 12, wobei die Zelle wie in Anspruch 8 oder Anspruch 9 definiert ist.

14. Verwendung einer Zelle nach einem der Ansprüche 8 bis 10 zur Herstellung eines Medikaments zur Transplantation zur Behandlung einer Erkrankung, die auf einem Zellverlust oder einer Zellschädigung beruht.

## Revendications

1. Construction comprenant un polynucléotide, qui code un oncogène inductible de manière conditionnelle, lié de manière fonctionnelle à l'élément amplificateur du deuxième intron du gène de la nestine, ou d'un fragment fonctionnel de celui-ci.

2. Construction selon la revendication 1, dans laquelle l'élément est un élément de 714 pb ou de 374 pb de la partie 3' du deuxième intron du gène de la nestine humaine.

3. Construction selon la revendication 1, dans laquelle l'oncogène est un c-myc ou le mutant sensible à la température du grand antigène T de SV 40.

4. Construction selon l'une quelconque des revendications précédentes, comprenant en outre un polynucléotide qui agit comme promoteur de l'oncogène.

5. Construction selon la revendication 4, dans laquelle le promoteur est le promoteur tk de HSV.

6. Construction selon l'une quelconque des revendications précédentes, comprenant en outre un polynucléotide qui code la sous-unité catalytique du complexe de télomérase.

7. Construction selon l'une quelconque des revendications 1 à 5, comprenant en outre un oncogène c-myc.

8. Cellule isolée comprenant une construction selon l'une quelconque des revendications précédentes.

9. Cellule isolée selon la revendication 8, qui est une cellule pluripotente indifférenciée mammalienne.

10. Cellule selon la revendication 8 ou 9, qui est une cellule neuroépithéliale pluripotente indifférenciée humaine.

11. Cellule selon l'une quelconque des revendications 8 à 10, pour un usage thérapeutique.

12. Utilisation d'une construction selon l'une quelconque des revendications 1 à 7, pour immortaliser de manière conditionnelle, une cellule *in vitro.*

13. Utilisation selon la revendication 12, dans laquelle la cellule est telle que définie à la revendication 9 ou à la revendication 10.

14. Utilisation d'une cellule selon l'une quelconque des revendications 8 à 10, dans la préparation d'un médicament pour la transplantation, pour traiter une maladie causée par une perte ou un dommage cellulaire.
